# FASCICULE DE BREVET EUROPEEN

(11) **EP 2 040 632 B1**
(45) Date de publication et mention de la délivrance du brevet: **05.05.2010**
(21) Numéro de dépôt: 06778770.5
(22) Date de dépôt: 03.07.2006
(51) Int. Cl.: A61B 17/70

(54) **SYSTEME DE STABILISATION INTER-EPINEUX**
INTERSPINÖSES STABILISIERUNGSSYSTEM
INTERSPINOUS STABILIZATION SYSTEM

(43) Date de publication de la demande: 01.04.2009
(73) Titulaire: Khalife, Sami, 80090 Amiens (FR)
(72) Inventeur: Khalife, Sami, 80090 Amiens (FR)
(74) Mandataire: Gasquet, Denis
(86) Numéro de dépôt international: PCT/FR2006/001589
(87) Numéro de publication internationale: WO 2008/003835

(56) Documents cités:
- FR-A1- 2 775 183
- US-A1- 2004 106 995
- US-A1- 2005 203 512

## Description

La présente invention concerne un matériel destiné à être implanté au niveau des vertèbres entre les apophyses épineuses pour améliorer la stabilité intervertébrale(en remettant sous tension l'appareil capsullo-ligamentaire, élargissant les trous de conjugaison et diminuant la pression dans l'espace du disque et entre les facettes articulaires) et éviter, à chaque fois que cela est possible, l'arthrodèse dans les maladies dégénératives déstabilisantes et symptomatiques.

L'invention concerne plus particulièrement des perfectionnements à ce type d'implants. Plusieurs modèles d'implants inter-épineux de ce type existent actuellement, et, malgré les améliorations successives mises au point par les fabricants, aucun de ces implants ne donne une entière satisfaction.

Généralement les implants inter-épineux sont en monobloc, soit en métal, soit en matériaux rigides (peek, etc.), soit en matériaux souples (polyuréthane recouvert de polyester tissé ou polyester tissé plat enroulé en cylindre). Les modes de fixation sont conçus de telle façon qu'on peut difficilement fixer plus qu'un espace à la fois avec une tension de serrage difficile à évaluer constamment. Il en résulte soit une fixation trop rigide pouvant entraîner parfois une arthrodèse et tel n'est pas le but, soit une fixation insuffisante pouvant entraîner un desserrage à moyen terme avec un conflit douloureux entre l'implant et les épineuses.

L'implant selon l'invention permet de remédier efficacement au problème primordial de l'adaptation de l'implant à toutes les variantes anatomiques et physiologiques.

L'implant selon l'invention est composé de trois éléments : deux pièces d'ancrage épineux rigides pouvant être de formes variables identiques ou pas, et d'une pièce intermédiaire d'épaisseur et de consistance physique variables permettant de régler à volonté la hauteur et l'élasticité du montage. La pièce intermédiaire s'encastre entre les pièces d'ancrage épineux.

Selon une caractéristique de l'invention, les interfaces entre les pièces d'ancrage épineux et la pièce intermédiaire sont de formes plates ou cylindriques permettant au montage d'être fixe mobile en rotation tout en assurant une stabilité satisfaisante et durable.

Selon une autre caractéristique de l'invention, l'implant peut être fixé aux épineuses par un dispositif métallique perforant l'os transversalement, soit par un dispositif ligamentaire souple contournant les épineuses.

Selon une disposition avantageuse de l'invention, la forme de l'implant et les modes de fixation sont conçus de telle sorte que l'on puisse instrumenter deux espaces adjacents ou plus si nécessaire.

Ainsi, le système de stabilisation inter-épineux selon l'invention composé de trois éléments et est caractérisé en ce que deux de ses éléments appelés pièces d'ancrage épineux, se fixent aux apophyses épineuses supérieure et inférieure et le troisième élément appelé pièce intermédiaire s'interpose entre les deux autres pour assurer la distraction de l'espace inter-épineux et la mobilité du dispositif dans les trois plans de l'espace.

Selon une caractéristique complémentaire, les pièces d'ancrage épineux possèdent du coté opposé à l'épineuse des orifices de différentes formes, ou des plots dans lesquels s'encastre la pièce intermédiaire par des plots ou des orifices correspondants, pour assurer la mobilité du système.

Selon une autre caractéristique, la pièce intermédiaire destinée à s'interposer entre les pièces d'ancrage épineux est de différentes formes et hauteurs, munie ou non de perforations ou de plots, et fabriquée avec différents types de matériaux, pour faciliter les mouvements entre les différents éléments du dispositif.

Ajoutons que la pièce intermédiaire est par exemple de forme elliptique, qui s'encastre dans des orifices de même forme sur les pièces d'ancrage épineux, pour assurer un mouvement multidirectionnel au dispositif.

Notons aussi que la pièce intermédiaire est munie de plots cylindriques qui s'encastrent dans les orifices correspondants des pièces d'ancrage épineux.

Selon une autre caractéristique, les pièces d'ancrage épineux sont munies d'orifices cylindriques pour recevoir les plots cylindriques de la pièce intermédiaire formant ainsi un dispositif mobile en rotation.

Selon une variante, les pièces d'ancrage épineux sont munies d'orifices oblongs pour recevoir les plots cylindriques de la pièce intermédiaire formant ainsi un dispositif mobile en rotation et en glissement latéral.

Selon une autre variante d'exécution, la pièce intermédiaire est munie d'un orifice fileté antérieur pour faciliter la préhension et l'assemblage, et perforée de tunnels transversaux permettant la fixation du système par un dispositif ligamentaire souple et participer accessoirement à l'effet d'amortissement.

Ajoutons que la pièce intermédiaire est constituée d'une facette plane pour permettre les rotations et les translations antéropostérieures et latérales et d'une facette sphérique pour permettre les inclinaisons antérieures, postérieures et latérales. L'ensemble permettant une mobilité dans les trois plans de l'espace.

D'autres caractéristiques et avantages de l'invention se dégageront de la description qui va suivre en regard des dessins annexés qui ne sont donnés qu'a titre d'exemples non limitatifs.

Les figures 1 à 9 représentent les différentes pièces du système selon l'invention.

Les figures 1 à 3 illustrent un premier mode de réalisation dit "système mobile".
La figure 1 est une vue antérieure du système mobile en assemblage.
La figure 2 est une vue latérale du système mobile en assemblage.
La figure 3 est une vue en perspective des trois pièces du système mobile séparées.
Les figures 4 à 6 illustrent un deuxième mode de réalisation dit "système fixé '.

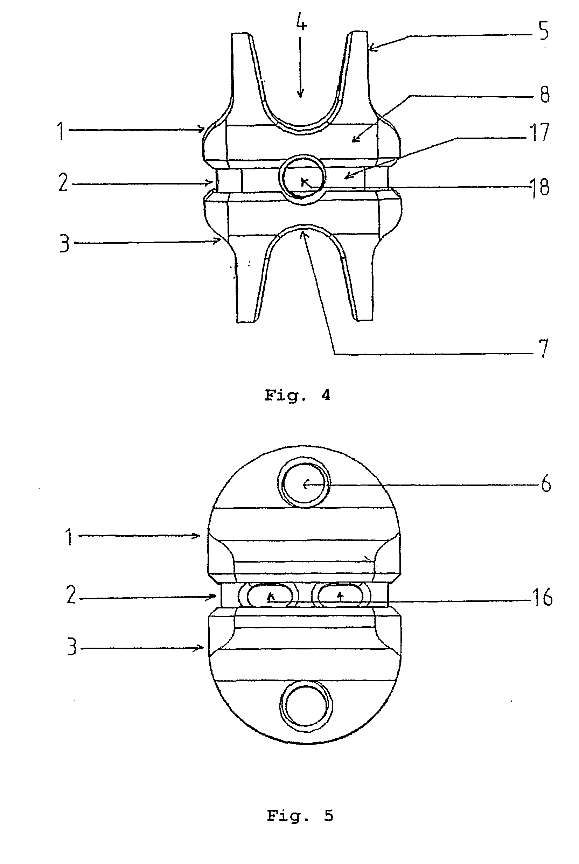

La figure 4 est une vue antérieure du système fixe.
La figure 5 est une vue latérale du système fixe.

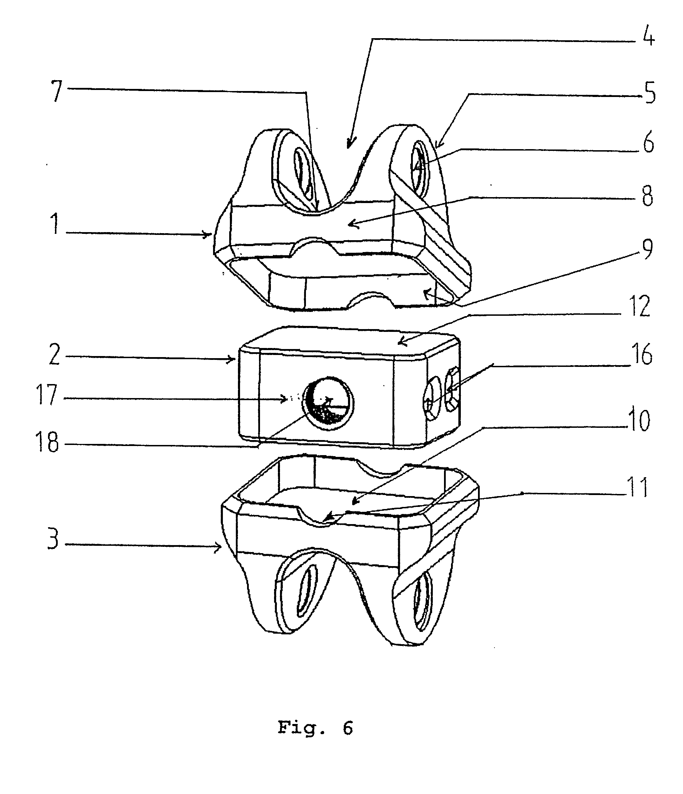

La figure 6 est une vue en perspective des trois pièces du système fixe.

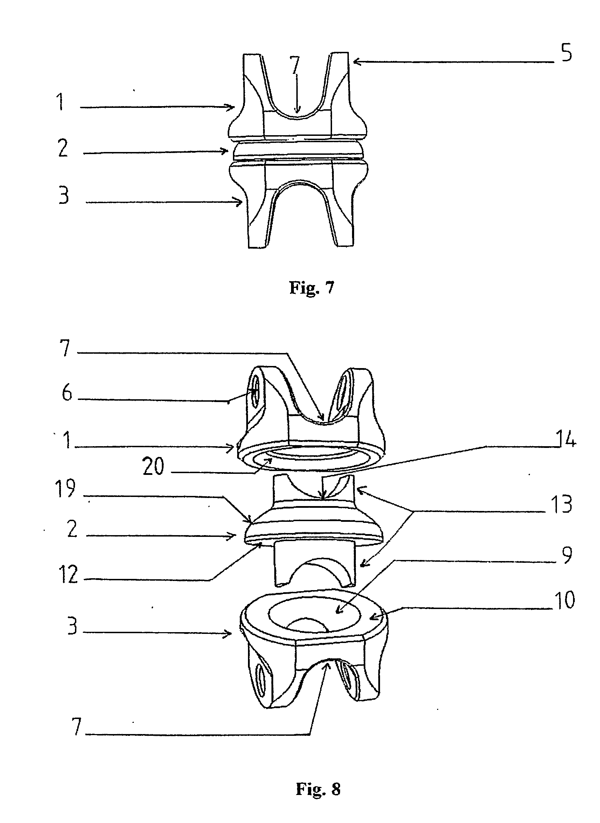

Les figures 7 à 9 illustrent un troisième mode de réalisation dit "système sphérique" .
La figure 7 est une vue antérieure du système sphérique
La figure 8 est une vue en perspective des trois pièces du système sphérique.

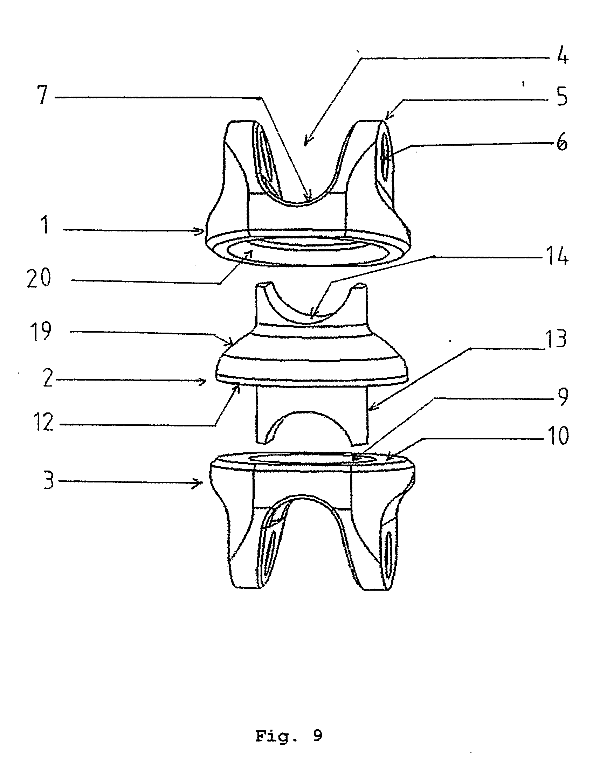

La figure 9 est une autre vue en perspective des trois pièces du système sphérique

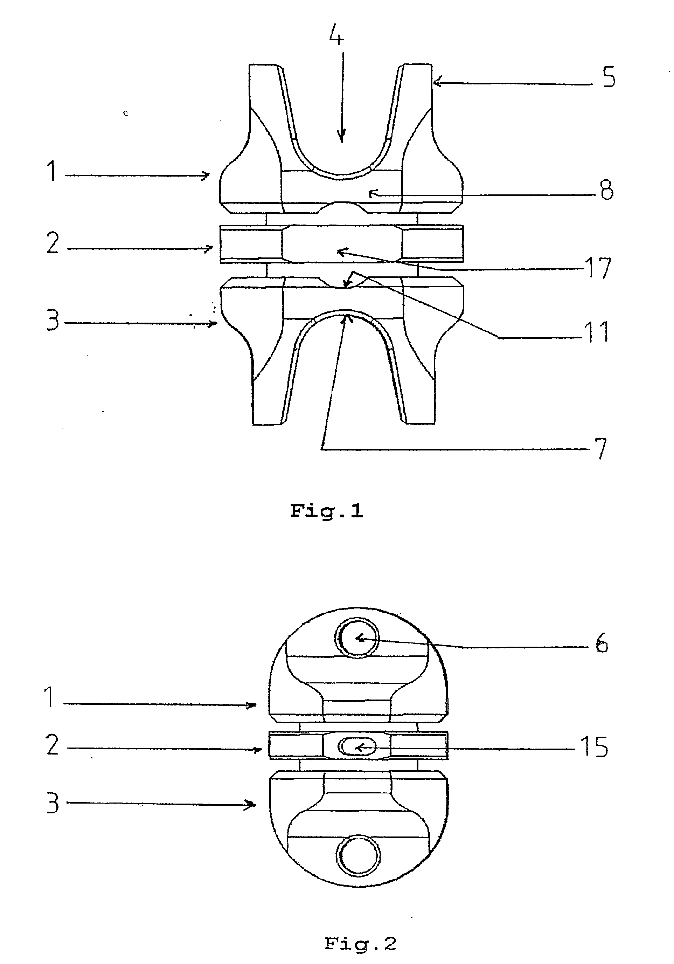

Les dessins annexés illustrent l'invention. On décrira ci-après, en faisant référence aux figures 1 à 9 les caractéristiques et le fonctionnement du « système de stabilisation inter-épineux. »

Le système selon l'invention est composé de trois éléments, deux pièces d'encrage (1, 3), à savoir une pièce d'ancrage épineux supérieure (1), une pièce intermédiaire (2) et une pièce d'ancrage épineux inférieure (3).

Selon une caractéristique de l'invention, tous les éléments sont usinés avec des matériaux biocompatibles métalliques ou non, et pouvant s'adapter à toutes les variantes anatomiques.

Selon une conception avantageuse, les pièces d'ancrage épineux (1, 3) sont constituées d'un corps en forme de gouttière (4) avec une gorge (7) de section cylindrique, des parois latérales évasées (5) percées de deux orifices (6) alignés horizontalement permettant la préhension et la fixation, à l'aide d'un dispositif métallique et dont les crêtes sont arrondies et émoussées. La base plane (10) des pièces d'ancrage épineux (1, 3) pour les deux premiers modes de réalisation, constitue l'interface avec la surface plane (12) de la pièce intermédiaire (2).

Selon une deuxième conception avantageuse, les pièces d'ancrage épineux (1, 3) possèdent, à leur base, des orifices (9) de différentes formes ou des plots dans lesquels viendra s'encastrer la pièce intermédiaire (2) par des plots (13) ou des orifices correspondants.

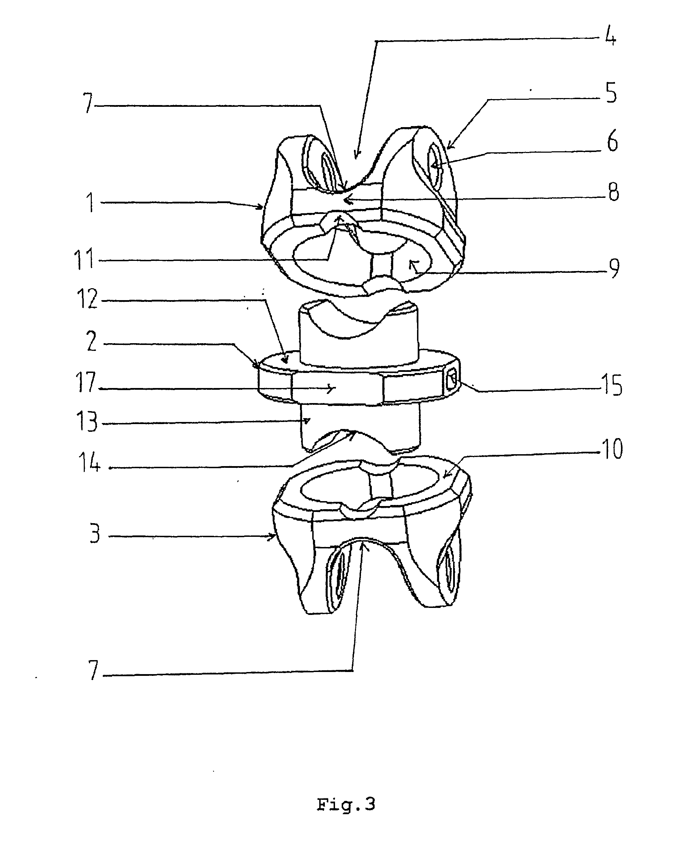

Selon une conception avantageuse les orifices (9) des pièces d'ancrage épineux peuvent avoir une section quadrangulaire pour le modèle fixe (fig.6), circulaire (fig.3) pour permettre des mouvements de rotation, ou oblongue pour permettre des mouvements de rotation et de glissement latéral.

Les pièces d'ancrage épineux (1, 3) possèdent des méplats (8) ou facettes, antérieurs et postérieurs avec une rainure médiane (11) facilitant l'alignement et l'assemblage.

Selon une autre caractéristique de l'invention, la pièce intermédiaire (2) destinée à s'interposer entre les pièces d'ancrage épineux (1, 3) peut être de différentes formes et hauteurs, munie ou non de plots (13) ou orifices.

Selon une caractéristique essentielle de l'invention, la pièce intermédiaire (2) peut être munie de plots cylindriques (13) qui s'encastrent dans les orifices cylindriques (9) des pièces d'ancrage épineux (1, 3) pour former un dispositif mobile en rotation. Quand les orifices (9) des pièces d'ancrage épineux (1, 3) sont oblongues le dispositif devient mobile en rotation et en glissement latéral.

Les extrémités des plots (13) de la pièce intermédiaire (2) comportent une échancrure (14) pour éviter tout conflit avec l'épineuse.

La pièce intermédiaire (2) peut être de forme parallélépipédique, avec des facettes d'encastrement plates (17) correspondant aux orifices de même formes (10) des pièces d'ancrage épineux (1, 3).

La pièce intermédiaire (2) peut avoir des caractéristiques mécaniques variables afin de donner un effet d'amortissement si nécessaire.

La pièce intermédiaire (2) est perforée de tunnels transversaux horizontaux (15, 16) permettant la fixation du système aux épineuses par un dispositif ligamentaire souple et de participer accessoirement à l'effet d'amortissement.

La pièce intermédiaire (2) peut être munie d'un orifice antérieur fileté (18) pour faciliter la préhension et l'assemblage.

Bien entendu, l'invention n'est pas limitée aux modes de réalisation décrits et représentés à titre d'exemples, mais elle comprend aussi tous les équivalents techniques ainsi que leurs combinaisons.

Ainsi, la figure 9, illustre une variante que l'on appellera système sphérique, selon laquelle la coopération entre la pièce intermédiaire (2) et la pièce d'encrage épineux supérieure (1) se fait grâce à un appui sphérique par coopération de la face d'appui (10) de la pièce intermédiaire te la face d'appui (20) de la pièce d'encrage (1) ces deux faces étant sphérique et en coopération de formes.

## Revendications

1. Système de stabilisation inter-épineux, **caractérisé en ce qu'**il est constitué de deux éléments appelés pièces d'ancrage épineux (1,3), à savoir une pièce d'ancrage supérieure (1) et une pièce d'ancrage inférieure (3), chacune des pièces d'ancrage étant constituée d'un corps en forme de gouttière pour être fixée aux apophyses épineuses respectivement supérieure et inférieure, tandis que le système comprend un troisième élément appelé pièce intermédiaire (2) qui s'interpose et s'encastre entre les deux pièces d'ancrage épineux pour assurer la distraction de l'espace inter-épineux et la mobilité du dispositif dans les trois plans de l'espace.

2. Système selon la revendication 1, **caractérisé en ce que** les pièces d'ancrage épineux (1, 3) possèdent du coté opposé à l'épineuse des orifices (9) de différentes formes, ou des plots dans lesquels s'encastre la pièce intermédiaire (2) par des plots (13) ou des orifices correspondants, pour assurer la mobilité du système.

3. Système selon la revendication 1 ou 2, **caractérisé en ce que** la pièce intermédiaire(2) destinée à s'interposer entre les pièces d'ancrage épineux (1, 3) est de différentes formes et hauteurs, munie ou non de perforations ou de plots (13), et fabriquée avec différents types de matériaux, pour faciliter les mouvements entre les différents éléments du dispositif.

4. Système selon l'une quelconque des revendications 1, 2 et 3, **caractérisé en ce que** la pièce intermédiaire(2) est de forme elliptique, qui s'encastre dans des orifices de même forme sur les pièces d'ancrage épineux (1, 3), pour assurer un mouvement multidirectionnel au dispositif.

5. Système selon l'une quelconque des revendications précédentes, **caractérisé en ce que** la pièce intermédiaire (2) est munie de plots cylindriques (13) qui s'encastrent dans les orifices correspondants (9) des pièces d'ancrage épineux (1,3).

6. Système selon l'une quelconque des revendications précédentes, **caractérisé en ce que** les pièces d'ancrage épineux (1, 3) sont munies d'orifices cylindriques (9) pour recevoir les plots cylindriques (13) de la pièce intermédiaire (2) formant ainsi un dispositif mobile en rotation.

7. Système selon l'une quelconque des revendications 1, à 5, **caractérisé en ce que** les pièces d'ancrage épineux (1, 3) sont munies d'orifices oblongs (9) pour recevoir les plots cylindriques (13) de la pièce intermédiaire (2)formant ainsi un dispositif mobile en rotation et en glissement latéral.

8. Système selon l'une quelconque des revendications précédentes, **caractérisé en ce que** la pièce intermédiaire (2) est munie d'un orifice fileté antérieur (18) pour faciliter la préhension et l'assemblage, et perforée de tunnels transversaux (15,16) permettant la fixation du système par un dispositif ligamentaire souple et participer accessoirement à l'effet d'amortissement.

9. Système selon l'une quelconque des revendications précédentes, **caractérisé en ce que** la pièce intermédiaire(2)est constituée d'une facette plane pour permettre les rotations et les translations antéropostérieures et latérales et d'une facette sphérique (19) pour permettre les inclinaisons antérieures, postérieures et latérales. L'ensemble permettant une mobilité dans les trois plans de l'espace.

## Claims

1. An interspinous stabilisation system, **characterised in that** it consists of two elements known as spinous anchoring components (1, 3), namely an upper anchoring component (1) and a lower anchoring component (3), each of the anchoring components consisting of a channel-shaped body provided for fixing to the respectively upper and lower spinous apophyses, while the system comprises a third element known as an intermediate component (2), which is interposed and fitted in between the two spinous anchoring components to ensure distraction of the interspinous space and mobility of the device in the three spatial planes.

2. A system according to claim 1, **characterised in that** the spinous anchoring components (1, 3) have differently shaped orifices (9) on the opposite side from the spinous process, or studs in which is fitted the intermediate component (2) by way of corresponding studs (13) or orifices, to ensure mobility of the system.

3. A system according to claim 1 or claim 2, **characterised in that** the intermediate component (2) designed to be interposed between the spinous anchoring components (1, 3) is of different shapes and heights, may or may not be provided with perforations or studs (13), and is made of different types of material, to facilitate movement between the different elements of the device.

4. A system according to any one of claims 1, 2 and 3, **characterised in that** the intermediate component (2) is elliptical in shape, fitting into orifices of the same shape in the spinous anchoring components (1, 3), to ensure multidirectional movement of the device.

5. A system according to any one of the preceding claims, **characterised in that** the intermediate component (2) is provided with cylindrical studs (13) which fit into the corresponding orifices (9) in the spinous anchoring components (1, 3).

6. A system according to any one of the preceding claims, **characterised in that** the spinous anchoring components (1, 3) are provided with cylindrical orifices (9) for receiving the cylindrical studs (13) on the intermediate component (2), so forming a rotationally mobile device.

7. A system according to any one of claims 1 to 5, **characterised in that** the spinous anchoring components (1, 3) are provided with oblong orifices (9) for receiving the cylindrical studs (13) of the intermediate component (2), so forming a rotationally mobile, laterally sliding device.

8. A system according to any one of the preceding claims, **characterised in that** the intermediate component (2) is provided with an anterior threaded orifice (18) for facilitating gripping and assembly, and is perforated by transverse tunnels (15, 16) allowing fixing of the system by a flexible ligamentary device and incidentally contributing to the shock-absorbing effect.

9. A system according to any one of the preceding claims, **characterised in that** the intermediate component (2) consists of a flat facet for allowing anterior-posterior and lateral, rotational and translational movements and a spherical facet (19) for allowing anterior, posterior and lateral inclination, the whole allowing mobility in the three spatial planes.

## Patentansprüche

1. Interspinöses Stabilisierungssystem, **dadurch gekennzeichnet, dass** es aus zwei Elementen gebildet ist, die spinale Verankerungsteile (1, 3) genannt werden, und zwar aus einem oberen Verankerungsteil (1) und aus einem unteren Verankerungsteil (3), wobei jedes der Verankerungsteile aus einem rinnenförmigen Körper gebildet ist, sodass es an dem oberen beziehungsweise unteren Dornfortsatz befestigt werden kann, während das System ein drittes Element umfasst, das Zwischenstück (2) genannt wird und das zwischen den beiden spinalen Verankerungsteilen angeordnet und eingepasst ist, um das Auseinanderdrücken des interspinösen Raumes und die Beweglichkeit der Vorrichtung in den drei Raumebenen zu gewährleisten.

2. System nach Anspruch 1, **dadurch gekennzeichnet, dass** die spinalen Verankerungsteile (1, 3) auf der Seite gegenüber dem Dornfortsatz Öffnungen (9) mit unterschiedlichen Formen oder Blöcke aufweisen, in die das Zwischenstück (2) über entsprechende Blöcke (13) oder Öffnungen passt, um die Beweglichkeit des Systems zu gewährleisten.

3. System nach Anspruch 1 oder 2, **dadurch gekennzeichnet, dass** das Zwischenstück (2), das dafür bestimmt ist, zwischen den spinalen Verankerungsteilen (1, 3) angeordnet zu werden, unterschiedlich geformt und unterschiedlich hoch ist, mit Löchern oder Blöcken (13) versehen ist oder nicht versehen ist und aus verschiedenen Arten von Materialien hergestellt ist, um die Bewegungen zwischen den verschiedenen Elementen der Vorrichtung zu erleichtern.

4. System nach einem der Ansprüche 1, 2 und 3, **dadurch gekennzeichnet, dass** das Zwischenstück (2) eine elliptische Form aufweist, die in ebenso geformte Öffnungen in den spinalen Verankerungsteilen (1, 3) passt, um eine Bewegung der Vorrichtung in mehreren Richtungen zu gewährleisten.

5. System nach einem der vorhergehenden Ansprüche, **dadurch gekennzeichnet, dass** das Zwischenstück (2) mit zylindrischen Blöcken (13) versehen ist, die In die entsprechenden Öffnungen (9) der spinalen Verankerungsteile (1, 3) passen.

6. System nach einem der vorhergehenden Ansprüche, **dadurch gekennzeichnet, dass** die spinalen Verankerungstelle (1, 3) mit zylindrischen Öffnungen (9) versehen sind, um die zylindrischen Blöcke (13) des Zwischenstücks (2) aufzunehmen und so eine drehbewegliche Vorrichtung zu bilden.

7. System nach einem der Ansprüche 1 bis 5, **dadurch gekennzeichnet, dass** die spinalen Verankerungsteile (1, 3) mit länglichen Öffnungen (9) versehen sind, um die zylindrischen Blöcke (13) des Zwischenstücks (2) aufzunehmen und so eine Vorrichtung zu bilden, die drehbeweglich und seitlich verschiebbar ist.

8. System nach einem der vorhergehenden Ansprüche, **dadurch gekennzeichnet, dass** das Zwischenstück (2) mit einer vorderen Öffnung (18) mit Gewinde versehen ist, um das Festhalten und die Montage zu erleichtern, und dass das Zwischenstück (2) mit Querkanälen (15, 16) versehen ist, damit das System mit einer flexiblen bandartigen Vorrichtung befestigt werden kann und mit denen zusätzlich zur stoßdämpfenden Wirkung beigetragen werden kann.

9. System nach einem der vorhergehenden Ansprüche, **dadurch gekennzeichnet, dass** das Zwischenstück (2) aus einer ebenen Fläche gebildet ist, um Drehbewegungen und geradlinige Bewegungen anteroposterior und zur Seite zu ermöglichen, und aus einer kugelförmigen Fläche (19), um Neigungsbewegungen nach vorn, nach hinten und zur Seite zu ermöglichen. Mit dieser Anordnung ist eine Beweglichkeit in den drei Raumebenen möglich.
